# EUROPEAN PATENT APPLICATION

(11) **EP 4 670 630 A1**
(43) Date of publication of application: **31.12.2025**
(21) Application number: 24185250.8
(22) Date of filing: 28.06.2024
(51) Int. Cl.: A61B 5/274, A61B 5/291, A61B 5/00

(54) **ELECTRODE ASSEMBLY FOR TRANSMITTING ELECTRIC SIGNALS FROM AND TO AN INDIVIDUAL**

(71) Applicant: Dätwyler Schweiz AG, 6467 Schattdorf (CH)
(72) Inventor: Segessenmann, Michael, 6468 Attinghausen (CH); Lucchini, Mattia Alberto, 6003 Luzern (CH)
(74) Representative: Prins Intellectual Property AG

(57) **Abstract**

An electrode assembly (1) for transmitting electric signals from or to an individual, the electrode assembly (1) comprising a support portion (2) for attaching the electrode assembly to a support arrangement and for providing electrical connection to a control unit and a contact portion (3) with a contact surface (31) for contacting an area of interest of the individual for transmitting electric signals; wherein the electrode assembly (1) has a contact side (12) facing the individual when applying the electrode assembly (1) to the individual, and a connector side (11) opposite the contact side (12); it is foreseen, that the electrode assembly (1) further comprises a motion suppression portion (4) made of electrically and/or ionically conductive elastomeric material for suppressing motion of the contact surface (21) relative to the area of interest of the individual during transmission of the electric signal, wherein the motion suppression portion (4) is arranged on the connector side (11) of the contact portion (3) and electrically connected to the contact portion (3).

## Description

### Technical Field

The invention relates to a soft and dry electrode assembly for detection of bioelectric signals in applications such as electroencephalography (EEG), electrocardiography (ECG), electrooculography (EOG) or electromyography (EMG) and/or transcutaneous or transcranial (i.e. non-invasive) electrical stimulation, such as transcranial Alternating Current Stimulation (tACS) or transcranial Direct Current Stimulation (tDCS).

### Technical Background

Bioelectrical signals are one of the most basic physiological signals of a human body. Soft and dry electrodes (SDE) or electrode assemblies - a type of bioelectric electrodes - are increasingly used for collecting bioelectric signals in applications such as electroencephalography (EEG), electrocardiography (ECG), electrooculography (EOG) or electromyography (EMG). Electrodes are also used for electrical stimulation, such as transcranial Alternating Current Stimulation (tACS) or transcranial Direct Current Stimulation (tDCS). Thus, electrodes are used to transmit electric signals from and to an individual.

In wearable devices comfort is a major focus, especially if the target market is the consumer one. Even if comfort is a subjective marker, it can be related to the pressure applied to the user body as consequence of wearing the device. The problem is common to all wearable devices, disregarding the design of devices and electrodes.

A common problem of any measurement or stimulation are so-called motion artifacts, which is something that may be observed in a scientific investigation or experiment that is not related to a real physiological phenomenon, but occurs as a result of the preparative or investigative procedure. Thus, motion artifacts are all kind of noises or interferences in a recorded signal that come from the electrode movement on a skin of an individual or electrode - skin contact imperfection. Thus, motion artifacts in bio-signal acquisition are essentially unwanted disturbances introduced into the signal due to the movement of the subject or the recording equipment.

So-called soft and dry electrodes (SDE) typically comprise an electrode body made from conductive elastomer and offer high wear comfort thanks to the softness of the material. At the same time, dry signal acquisition is possible without use of conductive gels, shortening preparation time and further increasing wear comfort. Because soft and dry electrodes adhere directly to the skin, noises cannot be reduced by using conductive gel. Motion artifacts can occur when the person moves or is in motion. In consequence, the (soft and dry) electrode might move relative to the skin of a person and thereby lose proper contact with the skin, which causes a significant deterioration in signal quality, making analysis and interpretation of the signal more difficult and less reliable. The effect can be particularly large when the electrode is attached to a more rigid structure, e.g. a headset. Thus, there is a need to reduce motion artifacts in electrode-based applications.

Different technical solutions are available to reduce the pressure perceived by the user (to increase comfort) and to reduce the motion artifacts, but they are rarely (if at all) combined in one solution and they normally involve additional engineering or complicated assembly procedures with multiple components (e.g. springs, foams, moving parts).

WO202326202 and WO202204408 each disclose a brain signal sensing headset with an electrode arrangement having a rather large and complex structure. The electrode may pivot in relation to a support structure of the headset so that the electrode makes full contact with the surface of a subject's head. The electrode arrangement does not compensate for lateral or radial movements. Motion artifacts are not reduced.

EP4014866 discloses an electrode arrangement with a rather large and complex structure. The electrode arrangement comprises an electrode member having several pins, a support shaft member for supporting the electrode member, a frame member for slidably holding the support shaft member in an axial direction thereof, and an elastic member in the form of a spring for biasing the electrode member toward the outside in the axial direction of the support shaft member. The electrode arrangement does not compensate for lateral or radial movements. Motion artifacts are not reduced.

EP3373802/WO2017083826 discloses an electrode arrangement where an electrode may pivot in relation to a support structure so that the electrode makes full contact with the surface of a subject's head. The support structure has three arms each provided with an electrode at its end. The support structure may be adjusted relative to a headset to place the electrodes at a desired position and is then locked. The electrode arrangement does not compensate for lateral or radial movements during measurements. Motion artifacts are not reduced.

Vasconcelos et al. [1] describes a dry electrode with arch-like contact structure, which are able to flex when applying a pressure. However, this concept cannot be used for other contact structures such as flat contact surfaces or pins.

### Literature:

[1] Vasconcelos et al., The Arch Electrode: A Novel Dry Electrode Concept for Improved Wearing Comfort, Front Neurosci. 2021; 15: 748100. doi: 10.3389/fnins.2021.748100

### Summary of the Invention

It is an objective of the invention to provide an electrode assembly for electric signal transmission showing reduced motion artefacts. At the same time, the electrode assembly should have a simple, compact and light design and be easily adaptable for different applications.

At least one of the objectives of the present invention is achieved by an electrode assembly for transmitting electric signals from or to an individual according to claim 1. The electrode assembly comprises a support portion for attaching the electrode assembly to a support arrangement and for providing electrical connection to a control unit and a contact portion with a contact surface for contacting an area of interest of the individual for transmitting electric signals. The electrode assembly has a contact side facing the individual when applying the electrode assembly to the individual and a connector side opposite the contact side. The electrode assembly further comprises a motion suppression portion made of electrically and/or ionically conductive elastomeric material for suppressing motion of the contact surface relative to the area of interest of the individual during transmission of the electric signal, wherein the motion suppression portion is arranged on the connector side of the contact portion and electrically connected to the contact portion.

In the context of the present invention "conductive elastomeric material" is understood as a "elastomeric material that has electrically or ionically conductive properties" to transmit a (bio-)electric signal from and to an individual.

The motion suppression portion is designed to deform under axial and/or lateral forces. Any motion of a support arrangement to which the electrode assembly is attached with the support portion and accordingly the support portion of the electrode assembly relative to the individual is suppressed by the motion suppression portion such that the relative motion is not propagated further to the contact portion. This controlled deformation will lead to improved comfort, i.e. reduced pressure on skin, better contact and reduced motion artifacts. The motion suppression portion can be applied to any type of rubber electrodes disregarding the design of the contact surface or support portion.

In the context of the present invention "upper" refers to the connector side and "lower" refers to the contact side of the electrode assembly. The electrode assembly has a "central" or "longitudinal axis" through all portions of the electrode assembly. "Radial" or "lateral" is used in the context of the central or longitudinal axis.

Further embodiments of the invention are set forth in the dependent claims.

In some embodiments the support portion may be at least partially made of electrically and/or ionically conductive elastomeric material. The support portion may comprise a non-elastomeric insert. The support portion may comprise a signal processing circuitry with an electric connector and could act as a so-called active electrode.

In some embodiments, the support portion may be a printed circuit board. The printed circuit board may comprise a signal processing circuitry with an electric connector. Thus, the electrode assembly may be a so-called active electrode.

In some embodiments the support portion may comprise attachment means for attaching the electrode assembly to the support arrangement and/or for providing electrical connection to the control unit. Thus, the attachment means may provide an electrical connection, or the support portion may comprise separate electrical connectors, e.g. in the case of an active electrode.

In some embodiments the attachment means may be an integral part formed by the support portion or an insert embedded in or over-moulded by the support portion. The insert may be a rigid connector insert. The attachment means may be formed as a snap-fit connector, preferably in the form of a snap fastener or snap button. In case that the attachment means are an integral part formed by the support portion, the support portion may be made of conductive elastomeric material.

In some embodiments the motion suppression portion may form an inner hollow space flanked by a circumferential side wall. The inner hollow space is typically centrally aligned with a central axis of the electrode assembly. The design with inner hollow space and circumferential side walls allows compression of the motion suppression portion along the central axis of the electrode assembly. The side walls may thereby bend laterally inwards or outwards. At the same time the lateral forces on the support portion may lead to a kind of tilting of the side walls thereby compensating a movement of the support portion relative to the contact portion. The side wall may be closed or may have openings.

In some embodiments the side wall may have a concave or convex shape as seen from the lateral outer side of the side wall. The concave or convex shape facilitates compression of the motion suppression portion. The convex shape may be formed by a side wall that bends outwards with the largest diameter near the middle of the motion suppression portion. The concave shape may be formed by a side wall that bends inwards with the smallest diameter near the middle of the motion suppression portion.

In some embodiments the side wall may have a circumferential shoulder, such that an upper or lower section of the motion suppression portion has a smaller diameter than the other section (lower or upper respectively) of the motion suppression portion.

In some embodiments the side wall may have an even thickness.

In some embodiments different sections of the side wall may have different thicknesses.

In some embodiments the contact portion may form a wall of the inner hollow space at the contact side of the motion suppression portion.

In some embodiments the support portion may form a wall of the inner hollow space at the connector side of the motion suppression portion.

In some embodiments the electrode assembly may be made of one or more parts, preferably two parts. The parts may be releasably connected to each other. A first part of the electrode assembly may form only the support portion or the support portion and the motion suppression portion, and a second part of the electrode assembly may form the contact portion and the motion suppression portion or only the contact portion, respectively.

In some embodiments the parts, in particular the first part and the second part of the electrode assembly may be provided with interlocking means for connecting the parts, in particular the first part and the second part of the electrode assembly in an electrically conductive manner.

In some embodiments the motion suppression portion may be provided with interlocking means for connecting the motion suppression portion in an electrically conductive manner to the support portion. The support portion may be a circuit board and the interlocking means may structurally and electrically connect the motion suppression portion with the contact portion to the circuit board.

In some embodiments the contact portion may have a planar, e.g. circular, contact surface. The contact portion may also comprise a base with several pins or legs forming the contact surface. Such pins or legs may be flexible, angled and/or comprise different shapes. Different pins or legs known in the art may be part of, arranged at or attached at the contact portion. Pins are typically designed to brush through hair and provide optimal contact to the individual. They cannot prevent motion artifacts. The contact portion is typically made of electrically and/or ionically conductive elastomeric material.

In some embodiments the contact portion and the motion suppression portion are formed in one piece.

The elastomeric material can be, for example, a synthetic or natural rubber, such as butyl rubber, isoprene rubber, butadiene rubber, halogenated butyl rubber (e.g., bromobutyl rubber), ethylene propylene terpolymer, silicone rubber, fluoro- or perfluoroelastomers, chlorosulfonate, polybutadiene, butyl, neoprene, nitrile, polyisoprene, buna-N, copolymer rubbers such as ethylene-propylene (EPR), ethylene-propylene-diene monomer (EPDM), acrylonitrile-butadiene (NBR or HNBR) and styrene-butadiene (SBR), blends such as ethylene or propylene-EPDM, EPR, or NBR, combinations thereof. The term "synthetic rubbers" also should be understood to encompass materials which alternatively may be classified broadly as thermoplastic or thermosetting elastomers such as polyurethanes, silicones, fluorosilicones, styrene-isoprene-styrene (SIS), and styrene-butadiene-styrene (SBS), as well as other polymers which exhibit rubber-like properties such as plasticized nylons, polyolefins, polyesters, ethylene vinyl acetates, fluoropolymers, and polyvinyl chloride.

The conductive properties of the elastomeric material may be achieved by adding conductive material. The conductive material may be carbon black, silver coated glass spheres, silver particles, Ag-coated aluminium beads, Ag-coated glass fibres, graphene, carbon nanotubes, graphite, stainless steel fibres, or any other suitable material.

### Brief Explanation of the Figures

The invention is described in greater detail below with reference to embodiments that are illustrated in the figures. The figures show:
- Fig. 1: a perspective view of a first variant of the electrode assembly;
- Fig. 2: a cross-sectional view of the electrode assembly of Fig. 1;
- Fig. 3: a perspective view of a second variant of the electrode assembly;
- Fig. 4: a cross-sectional view of the electrode assembly of Fig. 3.

### Embodiments of the Invention

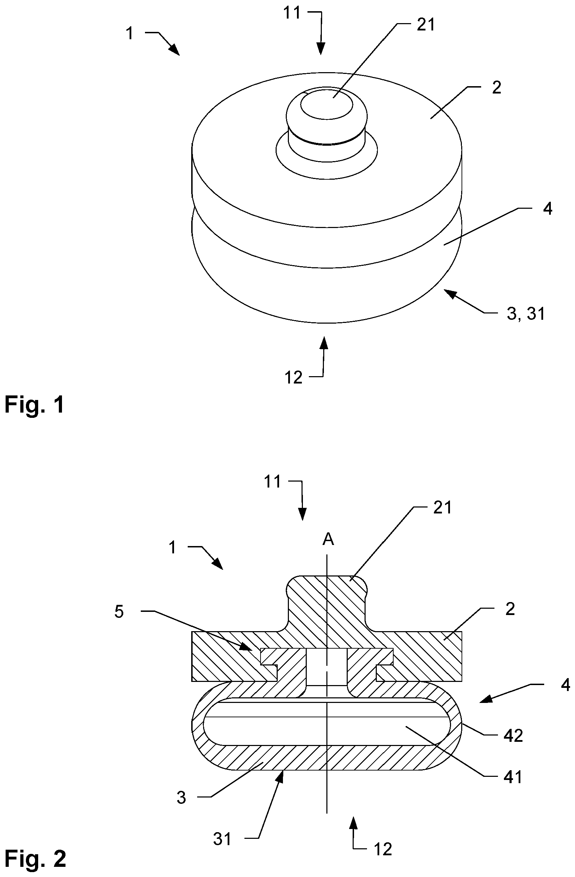

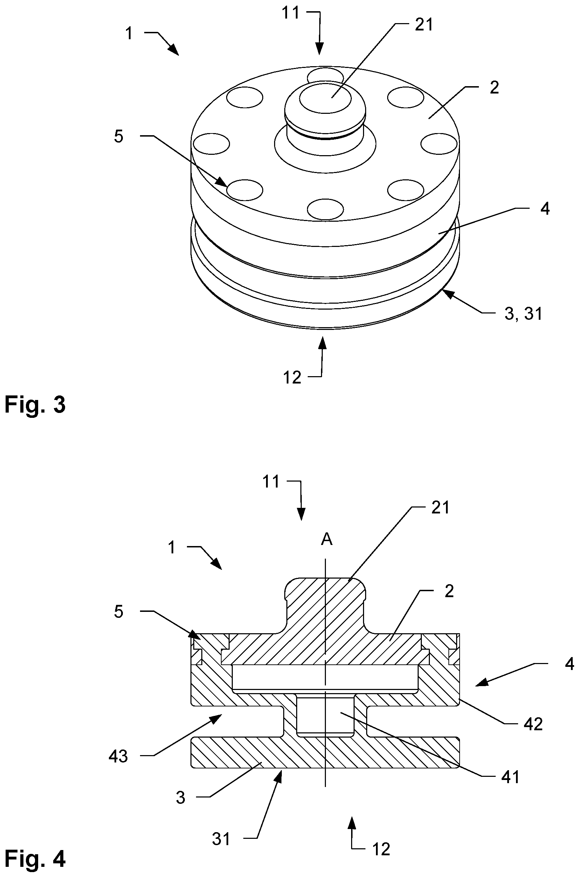

Figs 1 and 2 show a first variant and Figs 3 and 4 a second variant of an electrode assembly 1 for transmitting electrical signals from or to an individual. The electrode assembly 1 comprises a support portion 2, a contact portion 3 and a motion suppression portion 4 arranged between the support portion 2 and the contact portion 3.

The support portion 2 is provided with attachment means 21 for attaching the electrode assembly to a support arrangement and electrically connecting the electrode assembly 1 to a control unit or an electronic system. In the shown examples the attachment means 21 is formed as a snap-fit connector and is an integral part of the support portion 2. The contact portion 3 forms a contact surface 31 for contacting an area of interest of the individual for transmitting an electric signal.

The shown electrode assembly is a so-called soft and dry electrode made of conductive elastomeric material, has a connector side 11 on the side of the attachment means 21, and a contact side 12 opposite the connector side 11 facing the individual when applying the electrode assembly 1 to the individual.

The motion suppression portion 4 in between the support portion 2 and the contact portion 3 is designed with a flexible structure able to absorb axial and lateral forces and thereby suppressing motion of the contact surface 31 relative to the area of interest of the individual during measurement upon axial or lateral movements of the support portion 2 or the apparatus/arrangement to which the electrode assembly 1 is attached.

To suppress axial and lateral motion the motion suppression portion may form a hollow inner 41 space flanked by a circumferential wall 42.

The shown electrode assembly 1 is constructed in two parts. An upper part forms the support portion 2. A lower part forms the contact portion 3 and the motion suppression portion 4. The two parts are releasably connected to each other via interlocking means 5.

In the shown electrode assembly 1 the contact surface 31 is formed by a planar circular surface of the contact portion 3. The surface could also serve as base for several contact pins as explained above.

The support portion 2 can also be designed in many different ways as explained above. E.g. the support portion 2 may be a printed circuit board to which the motion suppression portion 4 is attached via the interlocking means 5.

In the first variant of Figs 1 and 2, the motion suppression portion 4 has the shape of a flattened ball or a cushion with a hollow inner space 41, which is open towards the connector side 11. The circumferential side wall 42 is bend outwards and has a convex shape.

The motion suppression portion 4 is further provided with interlocking means 5 on the connector side 11 for attachment to corresponding interlocking means 5 on the contact side of the support portion 2.

The relatively thin side wall 42 and the inner hollow space 41 of the motion suppression portion 4 allow a movement of the support portion 2 relative to the contact portion 3 thereby avoiding so-called motion artifacts.

The second variant of the electrode assembly shown in Figs 3 and 4 differs from the first variant in that the side wall 42 is differently shaped. The side wall 42 is provided with a circumferential shoulder 43 such that an upper cylindrical section of the side wall 42 has a larger diameter than a lower cylindrical section of the side wall 42. The lower section of the side wall 42 is formed to a disc-like contact portion 3. The upper section of the side wall 42 is provided with several interlocking means 5 for attachment to corresponding interlocking means 5 on the support portion 2. The step-like side wall allows for suppression of axial and lateral movement of the support portion 2 relative to the contact portion 3.

### Reference Signs

- 1: electrode assembly
- 11: connector side
- 12: contact side
- 2: support portion
- 21: attachment means
- 3: contact portion
- 31: contact surface
- 4: motion suppression portion
- 41: hollow space
- 42: lateral side wall
- 43: circumferential shoulder
- 5: interlocking means
- A: central axis

## Claims

1. Electrode assembly (1) for transmitting an electric signal from or to an individual, the electrode assembly (1) comprises
a support portion (2) for attaching the electrode assembly to a support arrangement and for providing electrical connection to a control unit and
a contact portion (3) with a contact surface (31) for contacting an area of interest of the individual for transmitting the electric signal;
wherein the electrode assembly (1) has
a contact side (12) facing the individual when applying the electrode assembly (1) to the individual, and
a connector side (11) opposite the contact side (12);
**characterized in that**
the electrode assembly (1) further comprises a motion suppression portion (4) made of electrically and/or ionically conductive elastomeric material for suppressing motion of the contact surface (21) relative to the area of interest of the individual during transmission of the electric signal, wherein the motion suppression portion (4) is arranged on the connector side (11) of the contact portion (3) and electrically connected to the contact portion (3).

2. Electrode assembly according to claim 1, wherein the support portion (2) is at least partially made of electrically and/or ionically conductive elastomeric material.

3. Electrode assembly according to claim 1, wherein the support portion (2) is a printed circuit board.

4. Electrode assembly according to one of the preceding claims, wherein the support portion (2) comprises attachment means (21) for attaching the electrode assembly to the support arrangement and/or for providing electrical connection to the control unit.

5. Electrode assembly according claim 4, wherein the attachment means (21) are an integral part formed by the support portion (2) or an insert embedded in or over-moulded by the support portion (2).

6. Electrode assembly according to one of the preceding claims, wherein the attachment means (21) are formed as a snap-fit connector, preferably in the form of a snap fastener or snap button.

7. Electrode assembly according to one of the preceding claims, wherein the motion suppression portion (4) forms an inner hollow space (41) flanked by a circumferential side wall (42).

8. Electrode assembly according to claim 7, wherein the side wall (42) has a concave or convex shape as seen from the lateral outer side of the side wall (42).

9. Electrode assembly according to claim 7, wherein the side wall has a circumferential shoulder (43).

10. Electrode assembly according to one of the claims 7 to 9, wherein the contact portion (3) forms a wall of the inner hollow space (41) at the contact side (12) of the motion suppression portion (4) and/or the support portion (2) forms a wall of the inner hollow space (41) at the connector side (11) of the motion suppression portion (4).

11. Electrode assembly according to one of the preceding claims, wherein the motion suppression portion (4) is provided with interlocking means (5) for connecting the motion suppression portion (4) in an electrically conductive manner to the support portion (2).

12. Electrode assembly according to one of the preceding claims, wherein the contact portion (3) has a planar contact surface (31) or is provided with several contact pins.

13. Electrode assembly according to one of the preceding claims, wherein the contact portion (3) is made of electrically and/or ionically conductive elastomeric material.

14. Electrode assembly according to one of the preceding claims, wherein the contact portion (3) and the motion suppression portion (4) are formed in one piece.
